# EUROPEAN PATENT SPECIFICATION

(11) **EP 1 563 821 B1**
(45) Date of publication and mention of the grant of the patent: **22.11.2006**
(21) Application number: 05010514.7
(22) Date of filing: 04.01.2001
(51) Int. Cl.: A61K 6/083, C08K 5/3435, C07D 211/94

(54) **Dental composition with light stability**
Dentalmasse mit Lichstabilität
Composition dentaire ayant une stabilité à la lumière

(30) Priority: 17.02.2000 US 183269 P
(43) Date of publication of application: 17.08.2005
(62) Divisional of application: 01901713.6
(73) Proprietor: DENTSPLY INTERNATIONAL, INC., York, PA 17405-0872 (US)
(72) Inventor: Walz, Uwe, 78465 Konstanz (DE); Klee, Joachim E., 78315 Radolfzell (DE)
(74) Representative: Hartz, Nikolai

(56) References cited:
- EP-A- 0 765 856
- US-A- 5 679 794
- US-A- 5 847 025
- US-A- 5 914 379

## Description

### Technical background

Dental compositions comprise polymerizable acrylates and/or methacrylates that are stabilized against spontaneous polymerization by using of free-radical scavenger such as the well-known phenols 2,6-di-tert.-butyl-4-cresol (BHT), hydroquinone or hydroquinone monomethylether (HQME). On the other side they contains a photoinitiator that must be react sensible to visible or UV- light to photoinitiate the free-radical polymerization.

Light curing dental materials mostly are applied under the conditions of relatively strong operating lamps. Consequently, the international standards require that a dental composite remains stable under an illumination of 10,000 lux for 60 seconds (ISO 4049), that a dental pit and fissure sealant and a light activated water based cement remains stable under an illumination of 8,000 lux for 25 seconds (ISO 6874) and for 30 s (ISO 9917-2), respectively.

To improve light stability an optimization of the initiator/inhibitor system leads to lengthening the working times under the conditions of a dental practice. However, this optimization is limited and leads to minor reduction of light sensitivity only.

Recently, it was found, that stable organic radicals reduce the light sensitivity of a dental light-curing composite material (N. Moszner, V. Rheinberger, US 5,847,025) when low molecular stable radicals such as 2,2-Diphenyl-1-picrylhydrazyl radicals, galvinoxyl radicals and/or triphenylmethyl radicals or 2,2,6,6-tetramethylpiperidin-1oxyl radicals are applied.

In the last decades dental composites becomes popularly as consequence of an improved dental supply. However, the application of this material class is combined with some new risks due to the release of parts of the composite, namely partly non-polymerized monomers (L. Shajii, J.P. Santerre, Biomaterials **20** (1999) 1897, W.R. Hume, T.M. Gerzia, Crit. Rev. Oral. Biol. Med. **7** (1996) 172) as well as portions of the inhibitors and/or initiator system (P.A. Liso et al., Biomaterials **18** (1997) 15). Furthermore, it is well known that free-radicals bearing some health risk (A.T. Diplock et al., Br. J. Nutr. **80** (1998), Suppl 1, 77; L.U. Thompson, Crit. Rev. Food Sci. Nutr. **34** (1994), 473).

Consequently, it seems desirable to use stable free-radicals for improved light sensitivity and to link them into the polymer system in order to avoid penetration and health risks.

The low molecular stable radicals that are suggested in US 5,847,025 bases on piperidinium 1-oxyl radicals bearing phenol or thiophenol groups or derivatives of carboxylic or thiocarboxylic acids.

### Description of the invention

Invented was a dental composition having an improved light and thermal stability, comprising a mixture of
(i) at least a polymerizable resin
(ii) at least a polymerizable monomer
(iii) at least a polymerization initiator and/or a sensitizer and stabilizer
(iv) at least an organic and/or inorganic filler and pigments in a content of 0 to 90 percent
(v) and at least one of the stable radicals of formulas 1 to 5 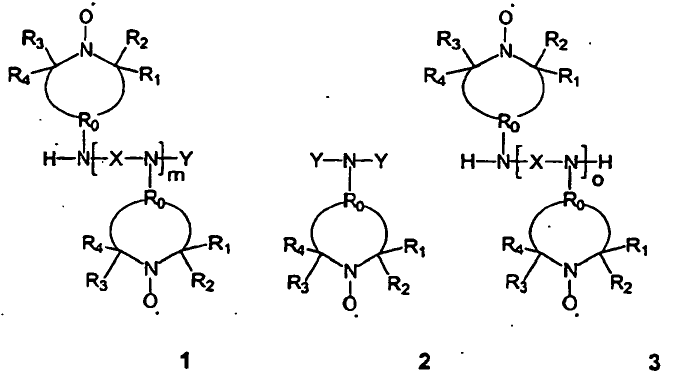 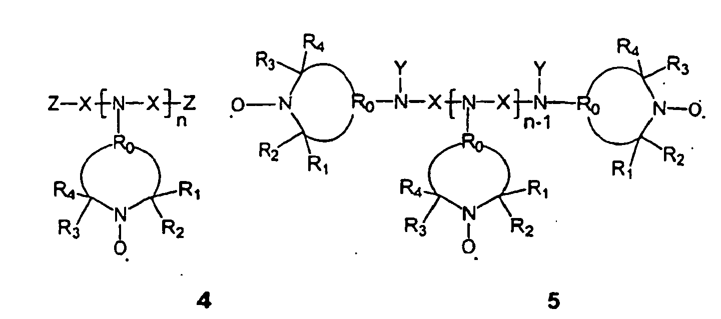
wherein
R₀ denotes a substituted or unsubstituted C₁ to C₁₈ alkylene,
R_{1'} R₂, R₃ and R₄ denotes a substituted or unsubstituted C₁ to C₁₈ alkylene, preferably a methyl group
X denotes a difunctional substituted or unsubstituted C₂ to C₃₀ alkylene, C₅ to C₃₀ substituted or unsubstituted cycloalkylene, substituted or unsubstituted C₅ to C₃₀ arylene or heteroarylene, preferably the following structures 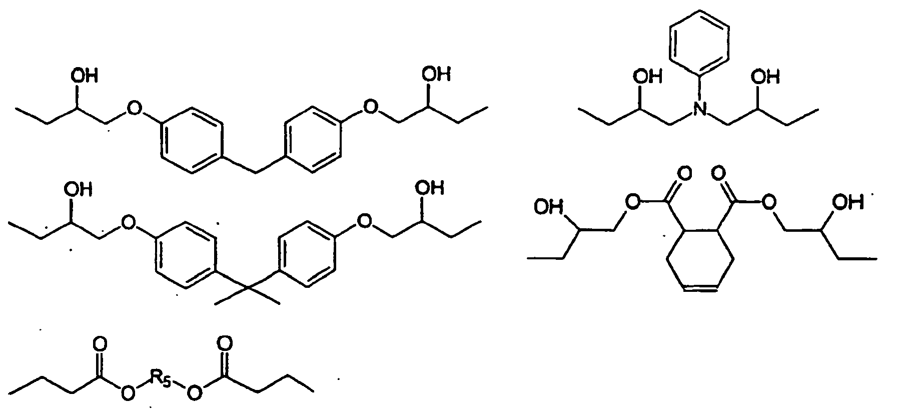 wherein R₅ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene,
Y denotes H or a monofunctional substituted or unsubstituted C₁ to C₁₈ alkyl, C₅ to C₁₈ substituted or unsubstituted cycloalkyl, substituted or unsubstituted C₅ to C₁₈ aryl or heteroaryl, preferably selected from the group 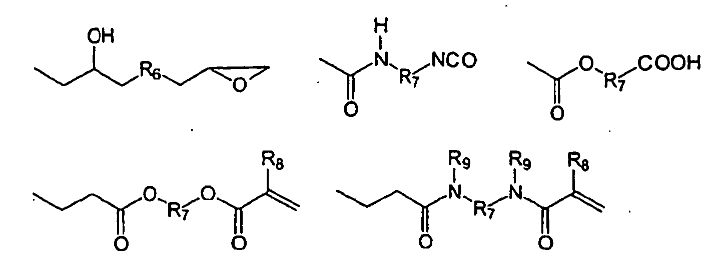 wherein
R₆ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably 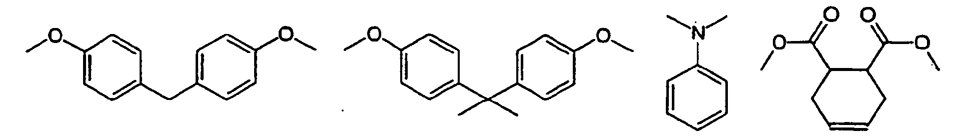 R₇ denotes difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably selected from the group
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
R₉ denotes a monofunctional substituted or unsubstituted C₁ to C₃₀ alkylene,
C₅ to C₃₀ substituted or unsubstituted cycloalkylene, substituted or unsubstituted C₅ to C₃₀ arylene or heteroarylene
Z denotes hydrogen, or a polymerizable moiety, preferably selected from the group of 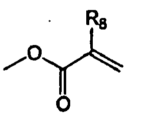 wherein
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
n, m and o are integers.

Preferably the dental composition comprises at least one of the compounds **6** to 10 which having at least one piperidinium nitroxyl radical moiety 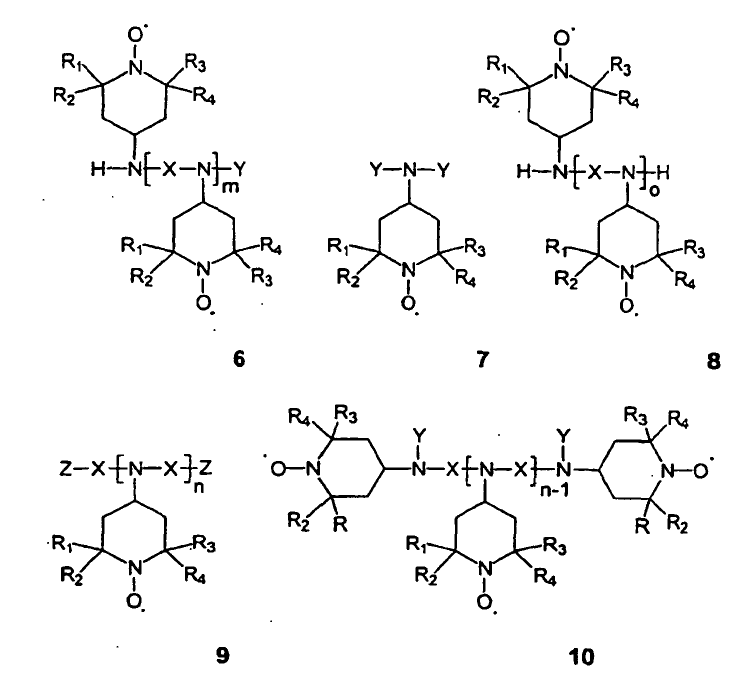 wherein
R₁, R₂, R₃ and R₄ denotes a substituted or unsubstituted C₁ to C₁₈ alkylene, preferably methyl group
X denotes a difunctional substituted or unsubstituted C₂ to C₃₀ alkylene, C₅ to C₃₀ substituted or unsubstituted cycloalkylene, substituted or unsubstituted C₅ to C₃₀ arylene or heteroarylene, preferably the following structures 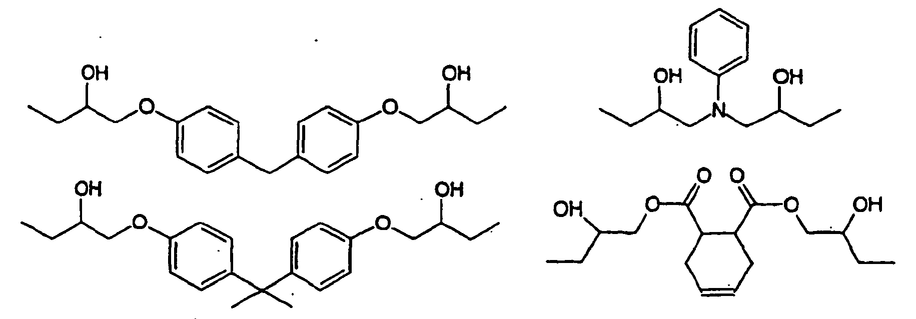 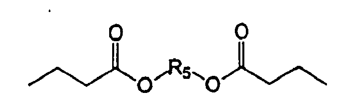 wherein R₅ denotes a difunctional substituted or unsubstituted C₁ to C₁₈ alkylene, C₅ to C₁₈ substituted or unsubstituted cycloalkylene, substituted or
unsubstituted C₅ to C₁₈ arylene or heteroarylene,
Y denotes H or a monofunctional substituted or unsubstituted C₁ to C₁₈ alkyl, C₅ to C₁₈ substituted or unsubstituted cycloalkyl, substituted or unsubstituted C₅ to C₁₈ aryl or heteroaryl, preferably selected from the group 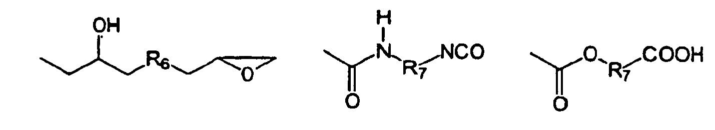 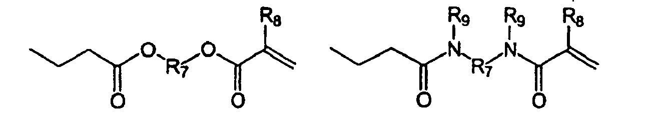 wherein
R₆ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably 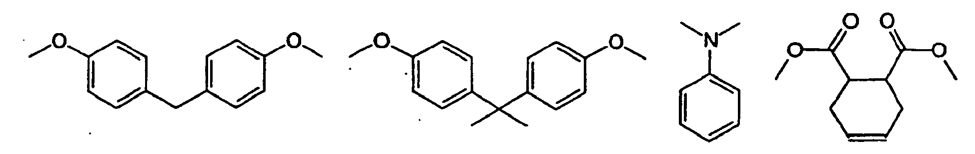 R₇ denotes difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably selected from the group
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
R₉ denotes a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
Z denotes hydrogen, or a polymerizable moiety, preferably selected from the 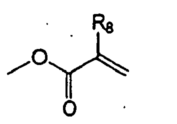 group of
wherein
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
n, m and o are integers.

The piperidinium nitroxyl radical moieties were obtained by two different pathways, namely by oxidation of the following compounds **11** to **15** or by incorporation of an amine comprising at least a nitroxyl radical moieties. 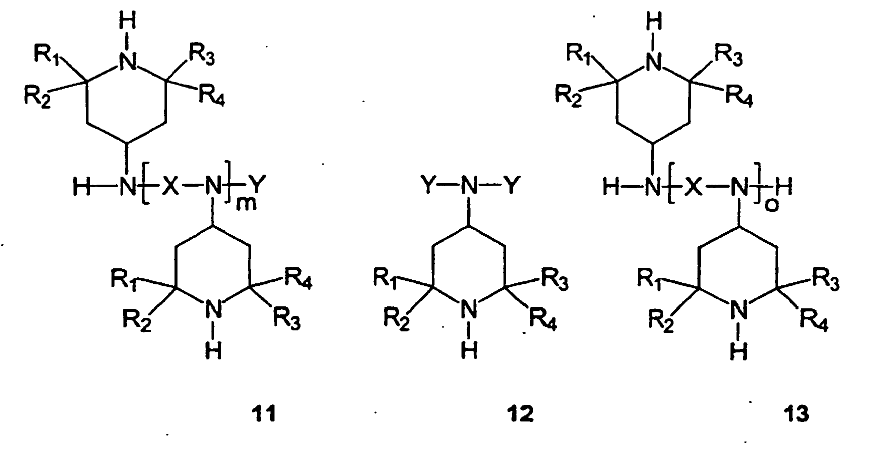 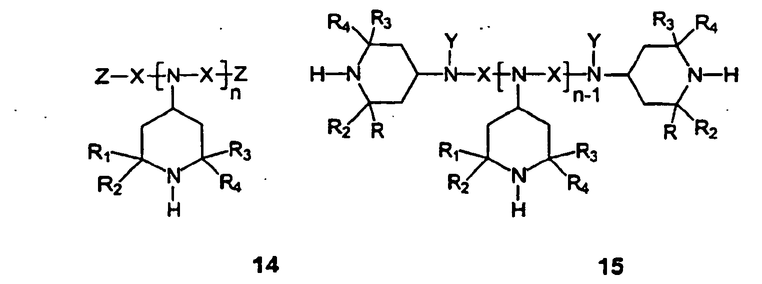 wherein
R_{1'} R₂, R₃ and R₄ denotes a substituted or unsubstituted C₁ to C₁₈ alkylene, preferably a methyl group
X denotes a difunctional substituted or unsubstituted C₂ to C₃₀ alkylene, C₅ to C₃₀ substituted or unsubstituted cycloalkylene, substituted or unsubstituted C₅ to C₃₀ arylene or heteroarylene, preferably the following structures 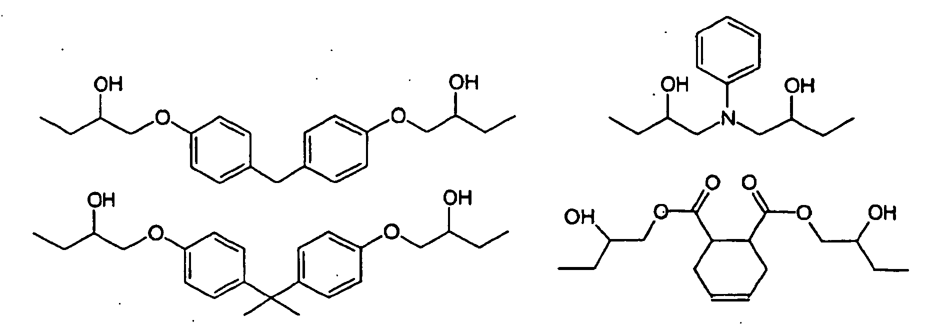 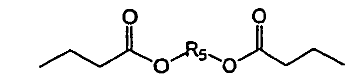 wherein R₅ denotes a difunctional substituted or unsubstituted C₁ to C₁₈ alkylene, C₅ to C₁₈ substituted or unsubstituted cycloalkylene, substituted or
unsubstituted C₅ to C₁₈ arylene or heteroarylene,
Y denotes H or a monofunctional substituted or unsubstituted C₁ to C₁₈ alkyl, C₅ to C₁₈ substituted or unsubstituted cycloalkyl, substituted or unsubstituted C₅ to C₁₈ aryl or heteroaryl, preferably selected from the group 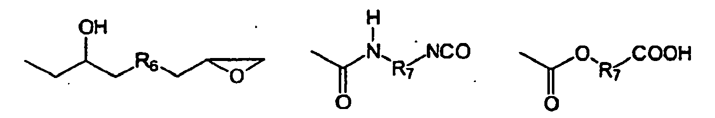 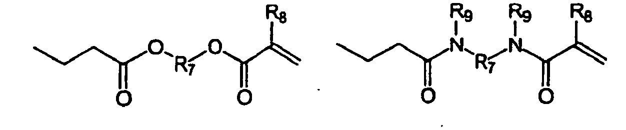 wherein
R₆ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably 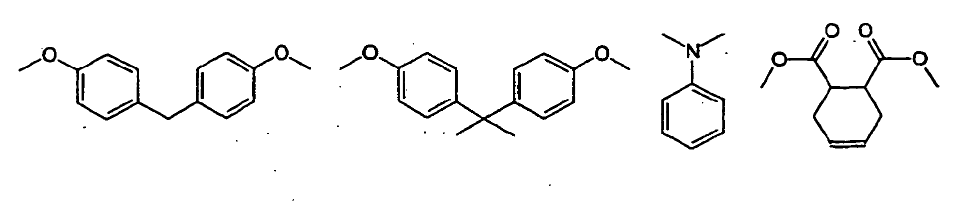 R₇ denotes difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably selected from the group
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
R₉ denotes a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
Z denotes hydrogen, or a polymerizable moiety, preferably selected from the 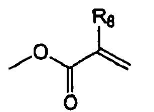 group of
wherein
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
n, m and o are integers.

Furthermore, polymers, prepolymers or macromonomers comprising at least a nitroxyl radical moieties were synthesized by direct incorporation of amines **16** comprising at least a nitroxyl radical moieties 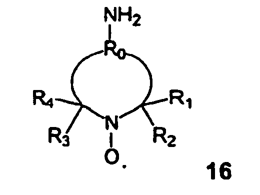 wherein
R₀ denotes a substituted or unsubstituted C₁ to C₁₈ alkylene,
R₁, R₂, R₃ and R₄ denotes a substituted or unsubstituted C₁ to C₁₈ alkylene, preferably methyl group
with a molecule of group A, selected from the group of a diepoxide, a diisocyanate, a dicarboxylic acid or a derivative thereof, a bisacrylamide or a bisacrylate or
with a molecule of group B, selected from the group of molecules that comprise at least an epoxide and a methacrylate group, an epoxide and an isocyanate, a methacrylate and an isocyanate group, an acrylate and a methacrylate group, or
with a mixture of molecules A and B.

Amines containing at least a nitroxyl radical moieties are used as comonomers for synthesis of polyamides, polyamidoamines, polyesteramines, polyureas, epoxide-amine addition polymers or prepolymers or macromonomers with the corresponding structural units mentioned above.

Preferably compounds 17 and 18 were use comprising a piperidinium nitroxyl radical moiety.

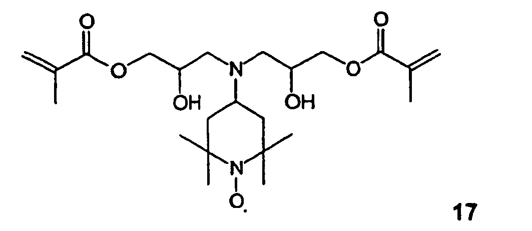

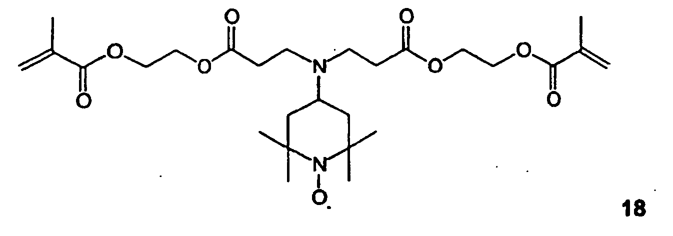

Surprisingly, the addition reaction of diepoxides and the steric hindered 4-amino-2,2,6,6-tetramethylpiperidin (ATMP) leads to linear soluble epoxide-amine addition polymers. The secondary amino groups do not react under the conditions of this polymerization. In the same manner the addition ATMP and Glycidylmethacrylat or Ethylene glycol acrylate methacrylate, respectively results in non-branched macromonomers.

Not less surprisingly it was found that the oxidation of prepolymers, macromonomers and polymers containing ATMP is possible without of a considerable degree of oxidation of hydroxylic moieties or methacrylic groups. The absorptions of hydroxylic groups at 3459/3421 cm⁻¹ and of the double bond at 1637 cm⁻¹ remains unchanged in the IR spectra compared to the non-oxidized molecules. Furthermore, no absorption of a keto group was observed.

The invented dental composition comprises stable radicals of formulas 1 to 5 in a content of 0.001 to 3.0 % by weight, preferably in a content of 0.01 to 1.0 % by weight and most preferably in a content of 0.1 to 0.5 % by weight.

For example a composite containing 2,2-Bis-[p-(2-hydroxy-3-methacryloyloxypropoxy)-phenyl]-propane, Triethyleneglycol dimethacrylate, UDMA, Camphor quinone and N,N-Dimethylaminoethylbenzoic acid ethylester and a Barium-alumo-silicate glass show a light sensitivity of 25 seconds at 10,000 lux. The compressive strength is 343.9 ± 7.3 MPa, the flexural strength (ISO 4049) is 119.2 ± 9.3 MPa and the E-modulus is 7802 ± 293 MPa.

A composite of the same composition that comprises additionally N,N-Bis-(2-hydroxy-3-methacryloyloxypropoxy)-4-amino-2,2,6,6-tetramethylpiperidin-1-oyxl radical of example 1 show a improved light sensitivity of 175 seconds at 10,000 lux.

### Example 1

### N,N-Bis-(2-hydroxy-3-methacryloyloxypropoxy)-4-amino-2,2,6,6-tetramethylpiperidin (GMA-ATMP)

4.998 g (35.17 mmol) Glycidylmethacrylat and 2.754 g (17.59 mmol) 4-amino-2,2,6,6-tetramethylpiperidin were homogeneously mixed and reacted for 48 hours at 80 °C. After that time the absorption of epoxide groups at 910 cm⁻¹ is completely missing.
Yield 7.756 g (100 % of th.)
C₂₃H₄₀N₂O₆, 440.58 g/mol
IR (cm⁻¹): 3421 (OH), 2975/2935 (CH₂/CH₃), 1726 (CO), 1637 (C=C) 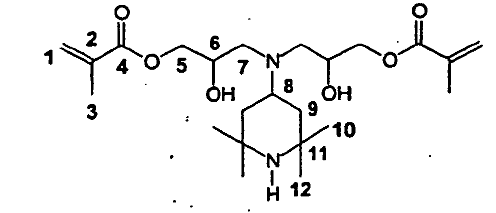 ¹³C NMR (ppm): 126.0 (1), 136.0 (2), 18.3 (3), 167.3 (4), 67.7 / 68.5 (5), 66.7 /67.1 (6), 63.1 (7), 54.0 / 54.2 (8), 51.3 / 51.8 (9), 41.3 (10), 28.4 / 28.5 (11), 35.2 (12)

### N,N-Bis-(2-hydroxy-3-methacryloyloxypropoxy)-4-amino-2,2,6,6-tetramethylpiperidin-1-oxyl radical (GMA-ATMPO)

In a three-necked flask equipped with a refluxer, a gas inlet pipe and a stirrer were dissolved 7.19 g (16.32 mmol) GMA-ATMP under stirring and heating to 60 °C. Then a stream of nitrogen was passed through this solution for 30 minutes.

In 250 ml Erlenmeyer flask were dissolved under stirring 8.06 g (24.48 mmol) K₃Fe(CN)₆ and 4.95 g (123.65 mmol) NaOH in 180 ml water.

Thereafter the aqueous solution was added to the three-necked flask and stirred intensively for 4 hours at 23 °C. The organic phase was separated and washed three times with 80 ml of deionized water and dried over Na₂SO₄. After removing the solvent at 50 °C and an end pressure of 3 mbar the products remains.

In the ESR spectrum a strong signal of nitroxyl radicals was found.
Yield 3.95 g (53.3 % of th.)
- IR (Sub.) cm⁻¹: vₛ(CH₃,CH₂) 2850: v(O-H) 3411; vₐₛ(CH³, CH²)2960, 29269;
v(C=O) 1716; v(C=C) 1637; v(C-O) 1173

### Example 2

### N,N-Bis-(2-hydroxy-3-methacryloyloxypropoxy)-4-amino-2,2,6,6-tetramethylpiperidin-1-oxyl radical (GMA-ATMPO)

1.6600 g (11.68 mmol) Glycidylmethacrylat and 1.0000 g (5.84 mmol) 4-amino-2,2,6,6-tetramethylpiperidin-1oxyl radical were homogeneously mixed and reacted 24 hours at 60 °C and 40 hours at 80 °C. After that time the absorption of epoxide groups at 910 cm⁻¹ is completely missing.

In the ESR spectrum a strong signal of nitroxyl radicals was found.
Yield 2.660 g (100 % of th.)
C₂₃H₃₉N₂O₇, 455.57 g/mol
IR (cm⁻¹): 3452 (OH), 2975/2935 (CH₂/CH₃), 1728 (CO), 1637 (C=C)

### Example 3

### Poly-[3,7-dihydroxy-1,9-dioxa-5-aza-(2,2,6,6-tetramethylpiperidine) nonamethylene-1,4-phenylene isopropylidene-1,4-phenylene] (AP-ATMP)

5.0000 g (14.69 mmol) Bis-2,2-[4-(2,3-epoxypropoxy)-phenyl]-propane (DGEBA) and 2.2953 g (14.69 mmol) 4-amino-2,2,6,6-tetramethylpiperidin were slightly heated to 60 °C and mixed homogeneously. Then the mixture was reacted at 60 °C for 24 hours. After that time the absorption of epoxide groups at 915 cm⁻¹ is completely missing.
Yield 7.295 g (100 % of th.)
(C₃₁H₄₆N₂O₄,)ₙ, (510.71)ₙ g/mol
¹³C NMR (ppm): 31.0 (1), 41.7 (2), 143.5 (3),127.7 (4), 113.9 (5),156.4 (6), 69.9 (7), 68.3 / 68.7 (8), 54.2 / 54.4 (9), 50.2 (10), 46.8 (11), 51.0 / 51.2 (12), 35.1 / 35.2 (13), 28.4 / 28.7 (14)

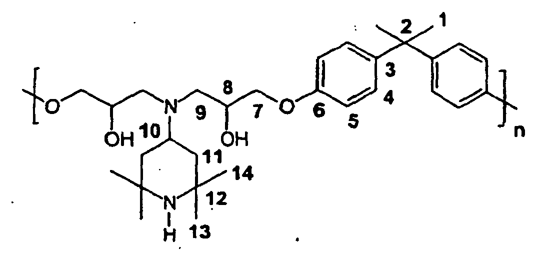

### Example 4

In a 250 ml three-necked flask equipped with a refluxer, a gas inlet pipe and a stirrer were dissolved 5.00 g (2.50 mmol) of the steric hindered amine Chimasorb 944 FD (CIBA-Geigy, CAS-Nr. 71878-19-8) in 200 ml Toluene under stirring and heating to 60 °C. Then a stream of nitrogen was passed through this solution for 30 minutes.

In 250 ml Erlenmeyer flask were dissolved under stirring 10.70 g (32.50 mmol) K₃Fe(CN)₈ and 6.57 g (164.16 mmol) NaOH in 80 ml water.

Thereafter the aqueous solution was added to the three-necked flask and stirred intensively for 4 hours at 23 °C. The organic phase was separated and washed three times with 80 ml of deionized water and dried over Na₂SO₄. After removing the solvent at 50 °C and an end pressure of 3 mbar the products remains.
Yield 4.33 g (86.60 % of th.)
In the ESR spectrum a strong signal of nitroxyl radicals was found.

### Example 5

### N,N-Bis-(3-oxa-4-oxo-6-methacryloyloxyhexyl)-4-amino-2,2,6,6-tetramethylpiperidin (AMA-ATMP)

10.000 g (63.99 mmol) 4-Amino-2,2,6,6-tetramethylpiperidin and 23.57 g (127.98 mmol) Ethylenglycol acrylatmethacrylat were homogeneously mixed and reacted at 23 °C for 14 days. After that time the absorption of acrylate double bond at 1620 cm⁻¹ is completely missing.
Yield 33.57 g (100 % of th.)
C₂₃H₄₀N₂O₆, 440.58 g/mol

### N,N-Bis-(3-oxa-4-oxo-6-methacryloyloxyhexyl)-4-amino-2,2,6,6-tetramethylpiperidin-1-oxyl radical (AMA-ATMPO)

N,N-Bis-(3-oxa-4-oxo-6-methacryloyloxyhexyl)-4-amino-2,2,6,6-tetramethylpiperidin was oxi-dized according the same procedure as described in example 1.
Yield 5.27 g (97.8 % of th.)
In the ESR spectrum a strong signal of nitroxyl radicals was found.

### Example 6

### N,N-Bis-(3-oxa-4-oxo-6-methacryloyloxyhexyl)-4-amino-2,2,6,6-tetramethylpiperidin-1-oxyl radical (AMA-ATMPO)

1.075 g (5.84 mmol) Ethylenglycol acrylatmethacrylat and 1.0000 g (5.84 mmol) 4-Amino-2,2 6,6-tetramethylpipeddin-1oxyl radical were homogeneously mixed and reacted 24 hours at 60 °C and 40 hours at 80 °C. After that time the absorption of acrylate double bond at 1620 cm⁻¹ is completely missing.

In the ESR spectrum a strong signal of nitroxyl radicals was found.
Yield 2.075 g (100 % of th.)
C₂₇H₄₃N₂O₉, 539.65 g/mol
IR (cm⁻¹): 2960/2845 (CH₂/CH₃), 1720 (CO), 1637 (C=C)

### Comparative Example 1

39.742 g 2,2-Bis-[p-(2-hydroxy-3-methacryloyloxypropoxy)-phenyl]-propane, 24.839 g Triethyleneglycol dimethacrylate, 34.774 g Urethane dimethacrylate, 0.298 g chamfer quinone and 0.348 g Dimethylaminoethyl benzoic acid ethylester were mixed homogeneously. To this resin mixture were added 270.370 g of a barium alumo-silicate glass and mixed homogeneously.

The properties are summarized in Table 1.

### Application Example 1

39.742 g 2,2-Bis-[p-(2-hydroxy-3-methacryloyloxypropoxy)-phenyl]-propane, 24.839 g Triethyleneglycol dimethacrylate, 34.774 g Urethane dimethacrylate, 0.298 g chamfer quinone, 0.348 g Dimethylaminoethyl benzoic acid ethylester and 0.034 g 4-Amino-2,2,6,6-tetramethyl-piperidin-1-oxyl radical (Fluka) were mixed homogeneously. To this resin mixture were added 270.370 g of a barium alumo-silicate glass and mixed homogeneously.

The properties are summarized in Table 1.

### Application Example 2

39.742 g 2,2-Bis-[p-(2-hydroxy-3-methacryloyloxypropoxy)-phenyl]-propane, 24.839 g Triethyleneglycol dimethacrylate, 34.774 g Urethane dimethacrylate, 0.298 g chamfer quinone, 0.348 g Dimethylaminoethyl benzoic acid ethylester and 0.091 g GMA-ATMPO of example 2 were mixed homogeneously. To this resin mixture were added 270.370 g of a barium alumo-silicate glass and mixed homogeneously.

The properties are summarized in Table 1.

### Application Example 3

39.742 g 2,2-Bis-[p-(2-hydroxy-3-methacryloyloxypropoxy)-phenyl]-propane, 24.839 g Triethyleneglycol dimethacrylate, 34.774 g Urethane dimethacrylate, 0.298 g chamfer quinone, 0.348 g Dimethylaminoethyl benzoic acid ethylester and 0.100 g AMA-ATMPO of example 5 were mixed homogeneously. To this resin mixture were added 270.370 g of a barium alumo-silicate glass and mixed homogeneously.

The properties are summarized in Table 1.

### Application Example 4

39.742 g 2,2-Bis-[p-(2-hydroxy-3-methacryloyloxypropoxy)-phenyl]-propane, 24.839 g Triethyleneglycol dimethacrylate, 34.774 g Urethane dimethacrylate, 0.298 g chamfer quinone, 0.348 g Dimethylaminoethyl benzoic acid ethylester and 0.100 g of oxidized amine of example 4 were mixed homogeneously. To this resin mixture were added 270.370 g of a barium alumo-silicate glass and mixed homogeneously.

The properties are summarized in Table 1.

**Table 1 Properties of dental composites of application examples 1 to 3 and of comparative example 1**

| Example | | Comp.1 | Appl. 1 | Appl.2 | Appl.3 |
|---|---|---|---|---|---|
| Sensitivity to ambient light,-ISO 4049 (10000 lux) | sec | 25 | 185 | 180 | 180 |
| Compressive strength | MPa | 343.9 ± 7.3 | 318.6 ± 17.8 | 316.3 ± 11.1 | 338.5 ± 6.6 |
| Flexural strength, ISO 4049 | MPa | 119.2 ± 9.3 | 107.7 ± 10.7 | 108.3 ± 5.0 | 117.9 ± 5.6 |
| E-modulus | MPa | 7802 ± 293 | 7691 ± 343 | 7324 ± 442 | 7698 ± 212 |

## Claims

1. Use of at least one of the stable radicals of formulas 1 to 5 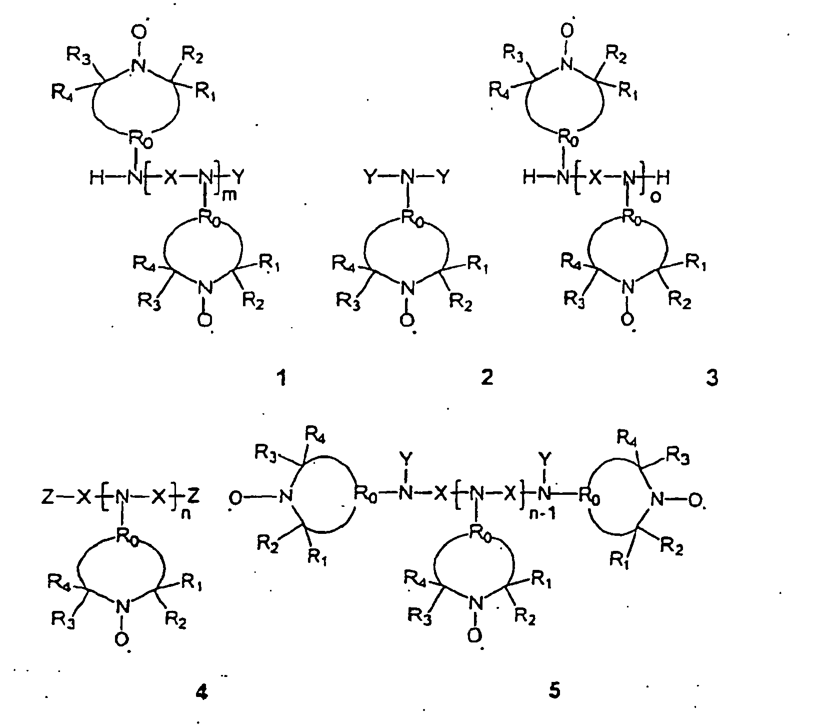 wherein
R₀ denotes a substituted or unsubstituted C₁ to C₁₈ alkylene,
R₁, R₂, R₃ and R₄ denotes a substituted or unsubstituted C₁ to C₁₈ alkyl;
X denotes a difunctional substituted or unsubstituted C₂ to C₃₀ alkylene, C₅ to C₃₀ substituted or unsubstituted cycloalkylene, substituted or unsubstituted C₅ to C₃₀ arylene or heteroarylene, preferably the following structures 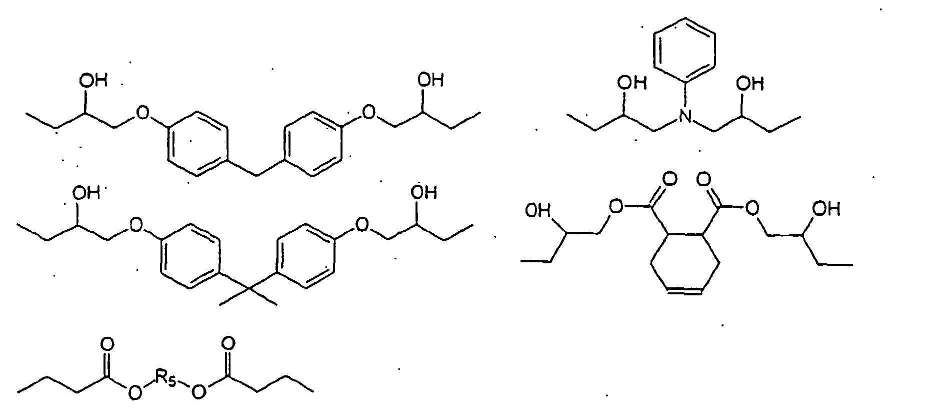
wherein
R₅ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene,
Y denotes H or amonofunctional substituted or unsubstituted C₁ to C₁₈ alkyl, C₅ to C₁₈ substituted or unsubstituted cycloalkyl, substituted or unsubstituted C₅ to C₁₈ aryl or heteroaryl, preferably selected from the group 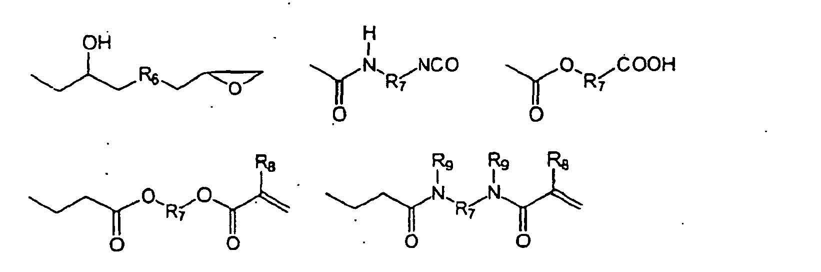
wherein
R₆ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably 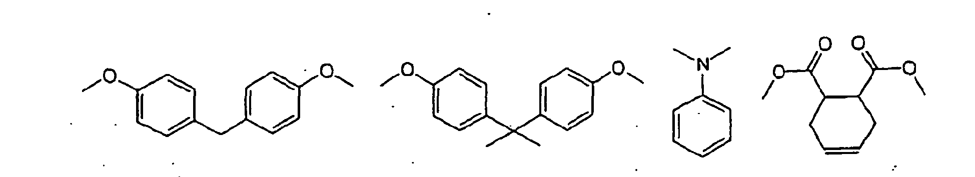
R₇ denotes difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene,
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
R₉ denotes a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
Z denotes hydrogen, or a polymerizable moiety, preferably selected from the group of 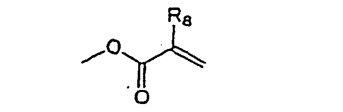
wherein
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene n, m and o are integers,
in the manufacture of a dental composition having light and thermal stability.

2. The use of claim 1, wherein the dental composition comprises at least one of the compounds 6 to 10 which has at least one piperidinium nitroxyl radical moiety 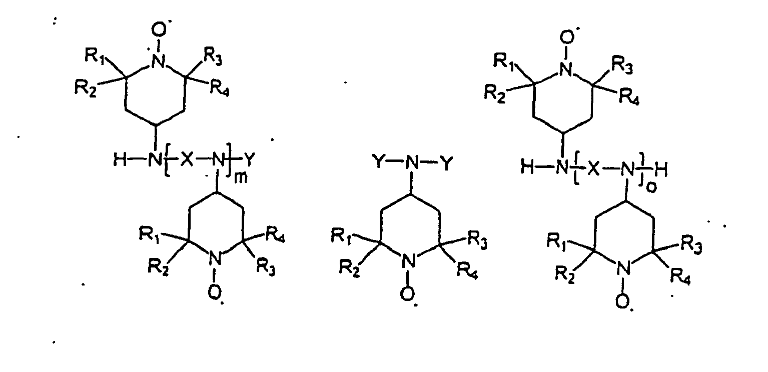 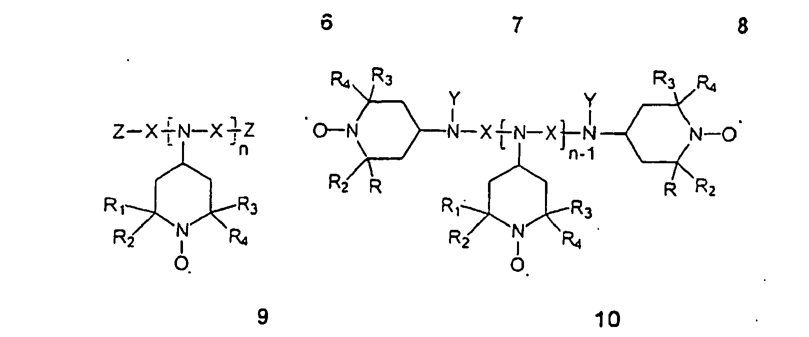 wherein
R₁, R₂, R₃ and R₄ denotes a substituted or unsubstituted C₁ to C₁₈ alkylene, preferably methyl group;
X denotes a difunctional substituted or unsubstituted C₂ to C₃₀ alkylene, C₅ to C₃₀ substituted or unsubstituted cycloalkylene, substituted or unsubstituted C₅ to C₃₀ arylene or heteroarylene, preferably the following structures 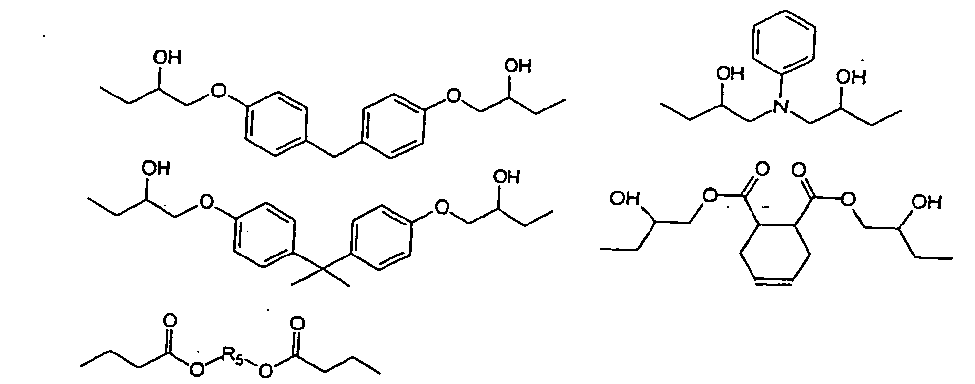
wherein
R₅ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene,
Y denotes H or a monofunctional substituted or unsubstituted C₁ to C₁₈ alkyl, C₅ to C₁₈ substituted or unsubstituted cycloalkyl, substituted or unsubstituted C₅ to C₁₈ aryl or heteroaryl, preferably selected from the group 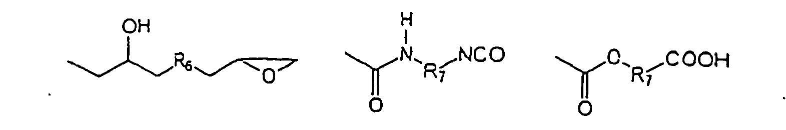 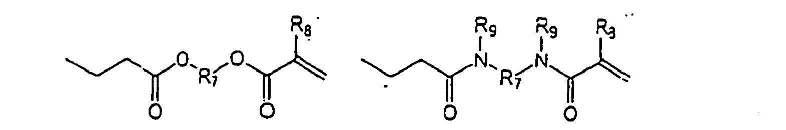
wherein
R₆ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably 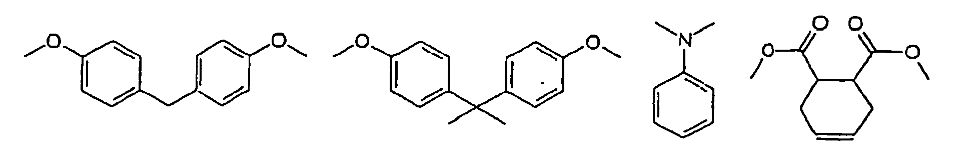
R₇ denotes difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene,
R₈ denotes H or a monofunctional substituted or unsubstituted C₁ to C₃₀ alkylene, C₅ to C₃₀ substituted or unsubstituted cycloalkylene, substituted or
R₉ unsubstituted C₅ to C₃₀ arylene or heteroarylene denotes a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene .
Z denotes hydrogen, or a polymerizable moiety, preferably selected from the group of 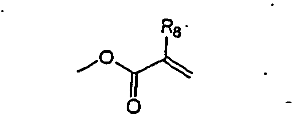
wherein
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene;
n, m and o are integers.

3. The use of claims 1 and 2, wherein the molecules containing piperidinium nitroxyl radical moieties are obtainable by oxidation of the following compounds 11 to 15 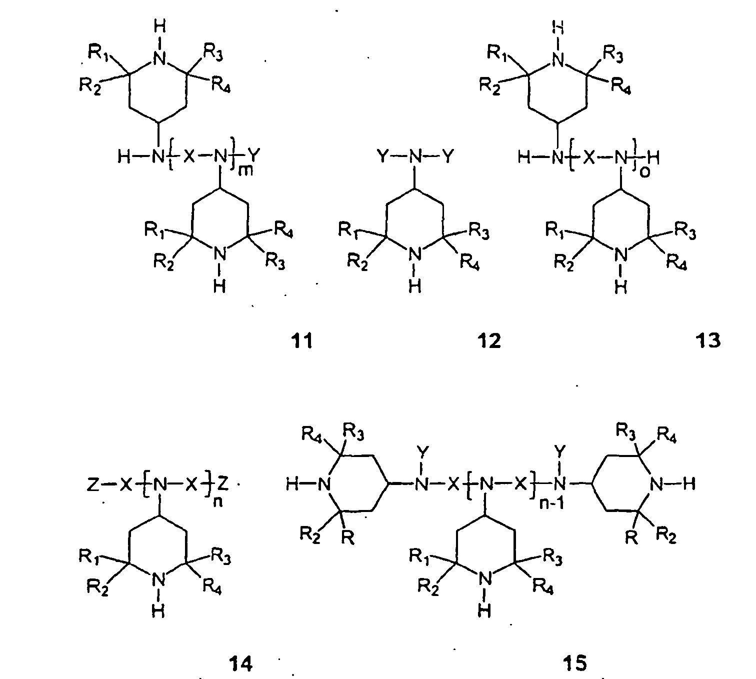 wherein
R₁, R₂, R₃ and R₄ denotes a substituted or unsubstituted C₁ to C₁₈ alkylene, preferably a methyl group
X denotes a difunctional substituted or unsubstituted C₂ to C₃₀ alkylene, C₅ to C₃₀ substituted or unsubstituted cycloalkylene, substituted or unsubstituted C₅ to C₃₀ arylene or heteroarylene, preferably the following structures 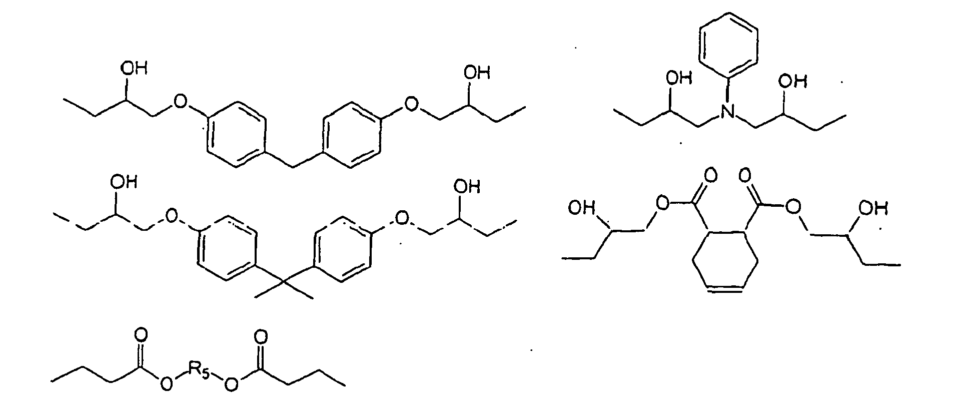
wherein
R₅ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene,
Y denotes H or a monofunctional substituted or unsubstituted C₁ to C₁₈ alkyl, C₅ to C₁₈ substituted or unsubstituted cycloalkyl, substituted or unsubstituted C₅ to C₁₈ aryl or heteroaryl, preferably selected from the group 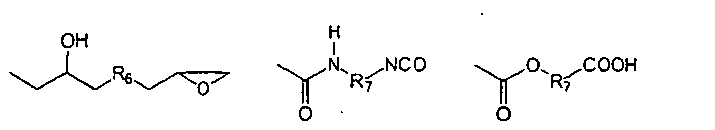 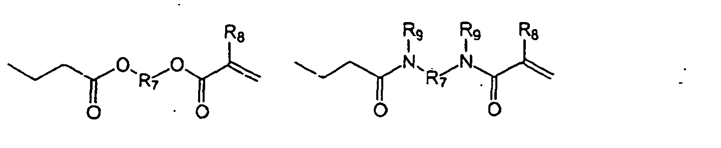
wherein
R₆ denotes a difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene, preferably 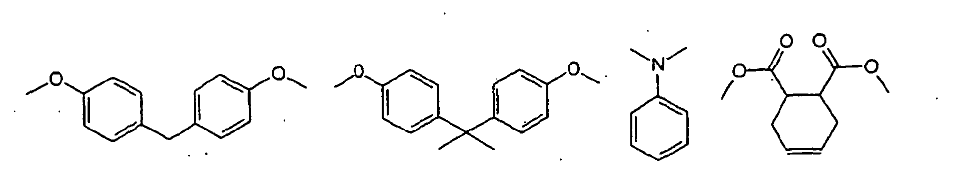
R₇ denotes difunctional substituted or unsubstituted C₁ to C₁₈ alkylene, C₅ to C₁₈ substituted or unsubstituted cycloalkylene, substituted or unsubstituted C₅ to C₁₈ arylene or heteroarylene,
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene
R₉ denotes a monofunctional substituted or unsubstituted C₁ to C₃₀ alkylene, C₅ to C₃₀ substituted or unsubstituted cycloalkylene, substituted or unsubstituted C₅. to C₃₀ arylene or heteroarylene
Z denotes hydrogen, or a polymerizable moiety, preferably selected from the group of
wherein 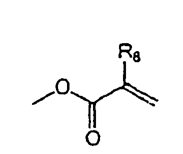
R₈ denotes H or a monofunctional substituted or unsubstituted C₁ to C₃₀ alkylene, C₅ to C₃₀ substituted or unsubstituted cycloalkylene, substituted or unsubstituted C₅ to C₃₀ arylene or heteroarylene n, m and o are integers.

4. The use of claim 1, wherein the dental composition comprising polymers, prepolymers or macromonomers containing nitroxyl radical moieties, which are obtainable by reaction of compound 16 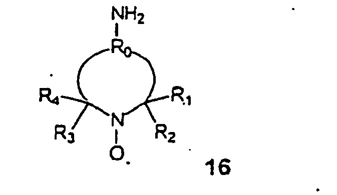 wherein
R₀ denotes a substituted or unsubstituted C₁ to C₁₈ alkylene, R₁, R₂, R₃ and R₄ denotes a substituted or unsubstituted C₁ to C₁₈ alkylene, preferably methyl group
with a molecule of group A, selected from the group of a diepoxide, a diisocyanate, a dicarboxylic acid or a derivative thereof, a bisacrylamide or a bisacrylate or
with a molecule of group B, selected from the group of molecules that comprise at least an epoxide and a methacrylate group, an epoxide and an isocyanate, a methacrylate and an isocyanate group, an acrylate and a methacrylate group, or
with a mixture of molecules A and B.

5. The use of claim 4, wherein amines containing nitroxyl radical moieties are contained as comonomers in polyamides, polyamidoamines, polyesteramines, polyureas, epoxide-amine addition polymers.

6. The use of claim 4, wherein amines containing nitroxyl radical moieties are contained as comonomers in macromonomers or prepolymers having polyamide, polyamidoamine, polyesteramine, polyurea or epoxide-amine addition polymer structural units.

7. The use of claim 1, wherein molecules containing piperidinium nitroxyl radical are compounds 17 and 18.

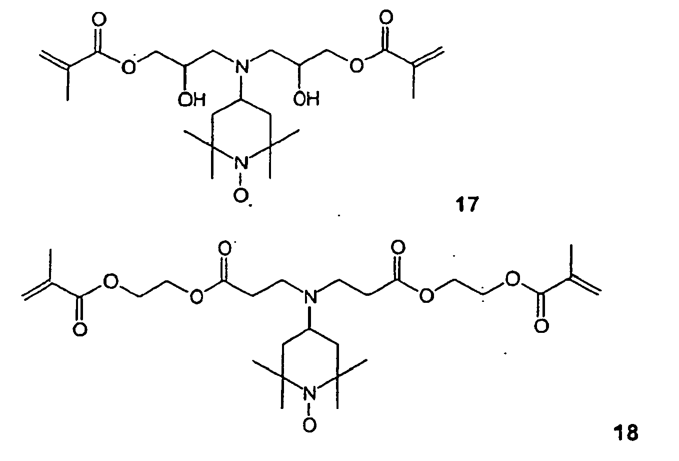

8. The use of of claim 1, wherein the dental composition comprises stable radicals of formulas 1 to 5 in a content of 0.001 to 3.0 % by weight.

9. The use of claim 1, wherein the dental composition comprises stable radicals of formulas 1 to 5 in a content of 0.01 to 1.0 % by weight.

10. The use of claim 1, wherein the dental composition comprises stable radicals of formulas 1 to 5 in a content of 0.01 to 0.2 % by weight.

11. Process for providing light and thermal stability to a dental composition, which comprises incorporating a prepolymer, macromonomer or polymer having a 4-amino-2,2,6,6-tetramethylpiperidin-1-oxyl moiety into the dental composition.

## Patentansprüche

1. Verwendung von mindestens einem der stabilen Radikalen der Formeln 1 bis 5 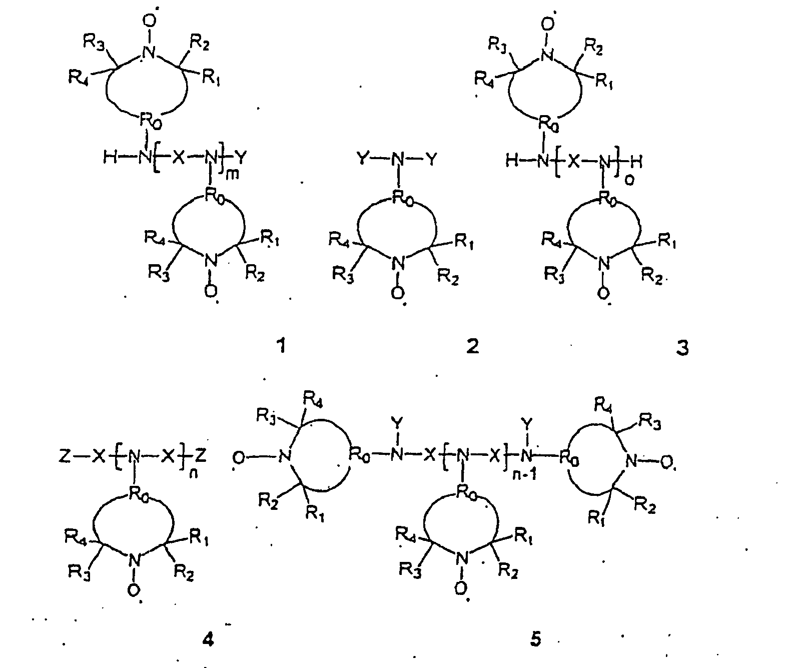 worin bedeuten
R₀ ein substituiertes oder unsubstituiertes C₁-C₁₈-Alkylen,
R₁, R₂, R₃ und R₄ ein substituiertes oder unsubstituiertes C₁-C₁₈-Alkyl;
X ein difunktionelles substituiertes oder unsubstituiertes C₂-C₃₀-Alkylen, C₅-C₃₀₋substituiertes oder unsubstituiertes Cycloalkylen, substituiertes oder unsubstituiertes C₅₋C₃₀-Arylen oder Heteroarylen, vorzugsweise die folgenden Strukturen 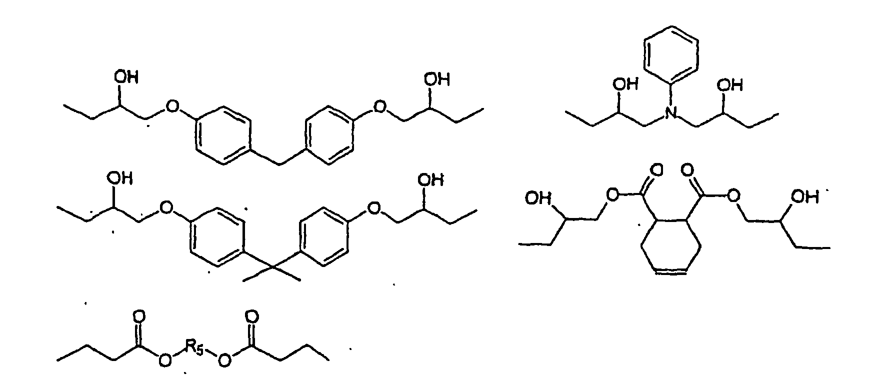
worin
R₅ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder-Heteroarylen bedeutet,
Y H oder ein monofunktionelles substituiertes oder unsubstituiertes C₁-C₁₈-Alkyl, C₅₋C₁₈-substituiertes oder unsubstituiertes Cycloalkyl, substituiertes oder unsubstituiertes C₅₋C₁₈-Aryl oder -Heteroaryl bedeutet, vorzugsweise ausgewählt aus der Gruppe 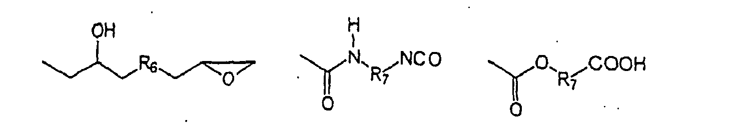 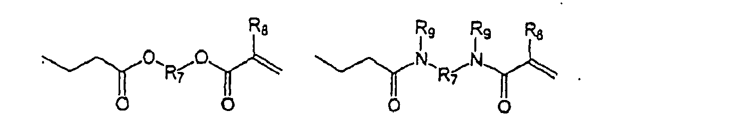
worin
R₆ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder -Heteroarylen bedeutet, vorzugsweise 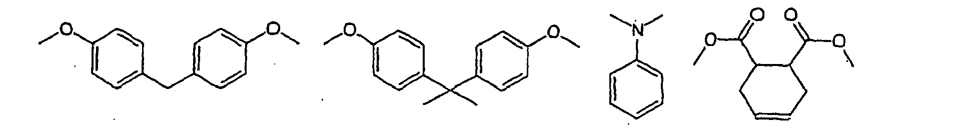
R₇ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder -Heteroarylen, bedeutet,
R₈ H oder ein monofunktionelles substituiertes oder unsubstituiertes C₁-C₃₀-Alkyl, C₅₋C₃₀-substituiertes oder unsubstituiertes Cycloalkylen, substituiertes oder unsubstituiertes C₅-C₃₀-Arylen oder -Heteroarylen bedeutet,
R₉ ein monofunktionelles substituiertes oder unsubstituiertes C₁-C₃₀-Alkyl, C₅-C₃₀₋substituiertes oder unsubstituiertes Cycloalkylen, substituiertes oder unsubstituiertes C₅₋C₃₀-Arylen oder -Heteroarylen, bedeutet,
Z Wasserstoff bedeutet oder eine polymerisierbare Einheit, vorzugsweise ausgewählt aus der Gruppe 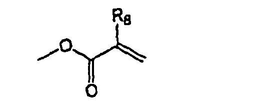
worin
R₈ H oder ein monofunktionelles substituiertes oder unsubstituiertes C₁-C₃₀-Alkyl, C₅₋C₃₀-substituiertes oder unsubstituiertes Cycloalkylen, substituiertes oder unsubstituiertes C₅-C₃₀-Arylen oder -Heteroarylen bedeutet, und n, m und o ganze Zahlen sind, zur Herstellung einer Dentalmasse, die Licht- und thermische Stabilität aufweist.

2. Verwendung nach Anspruch 1, worin die Dentalmasse mindestens eine der Verbindungen 6 bis 10 umfasst, die mindestens eine Piperidiniumnitroxylradikal-Einheit aufweist 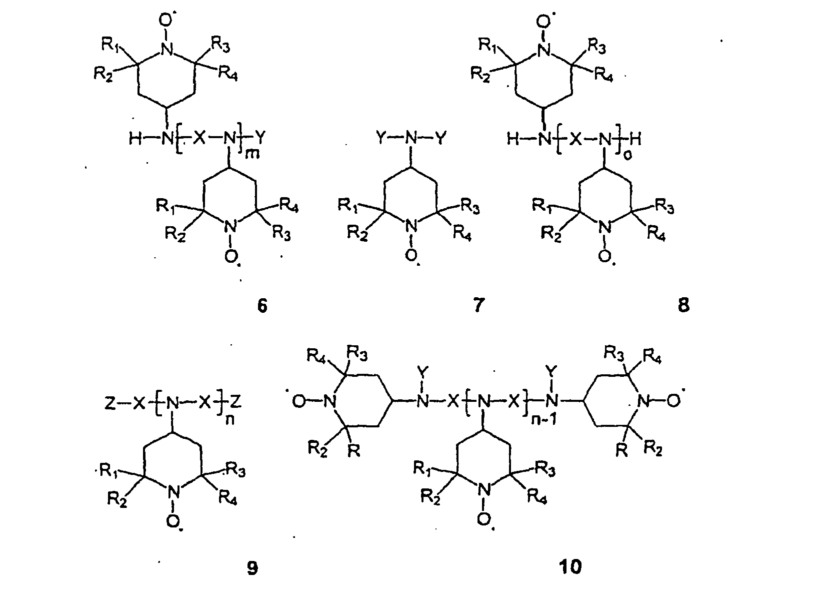 worin bedeuten
R₁, R₂, R₃ und R₄ ein substituiertes oder unsubstituiertes C₁-C₁₈-Alkyl, vorzugsweise eine Methylgruppe;
X ein difunktionelles substituiertes oder unsubstituiertes C₂-C₃₀-Alkylen, C₅-C₃₀₋substituiertes oder unsubstituiertes Cycloalkylen, substituiertes oder unsubstituiertes C₅₋C₃₀-Arylen oder -Heteroarylen, vorzugsweise die folgenden Strukturen 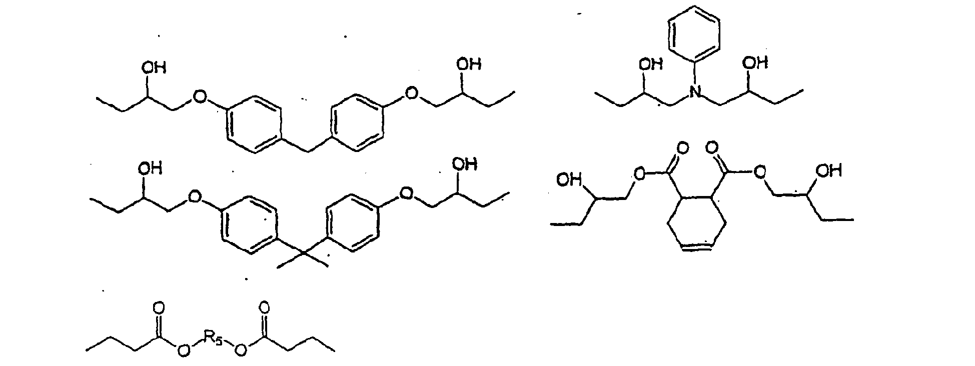
worin
R₅ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder -Heteroarylen bedeutet;
Y H oder ein monofunktionelles substituiertes oder unsubstituiertes C₁-C₁₈-Alkyl, C₅₋C₁₈-substituiertes oder unsubstituiertes Cycloalkyl, substituiertes oder unsubstituiertes C₅₋C₁₈-Aryl oder -Heteroaryl, vorzugsweise ausgewählt aus der Gruppe 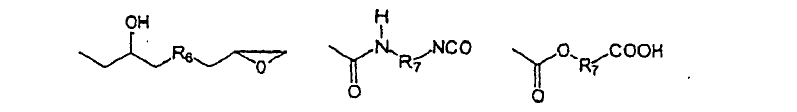 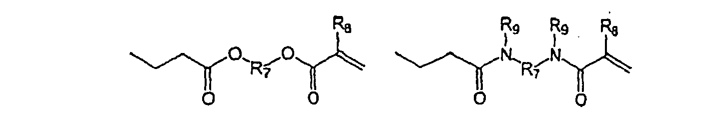
worin
R₆ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder -Heteroarylen bedeutet, vorzugsweise 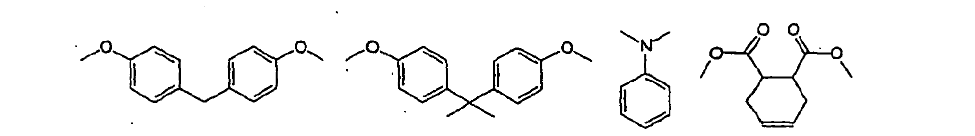
R₇ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder -Heteroarylen bedeutet,
R₈ H oder ein monofunktionelles substituiertes oder unsubstituiertes C₁-C₃₀-Alkyl, C₅₋C₃₀-substituiertes oder unsubstituiertes Cycloalkylen, substituiertes oder unsubstituiertes C₅-C₃₀-Arylen oder -Heteroarylen bedeutet,
R₉ ein monofunktionelles substituiertes oder unsubstituiertes C₁-C₃₀-Alkyl, C₅-C₃₀₋substituiertes oder unsubstituiertes Cycloalkylen, substituiertes oder unsubstituiertes C₅₋C₃₀-Arylen oder -Heteroarylen bedeutet,
Z Wasserstoff bedeutet, oder eine polymerisierbare Einheit, vorzugsweise ausgewählt aus der Gruppe 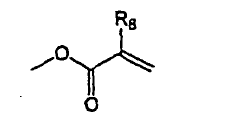
worin
R₈ H oder ein monofunktionelles substituiertes oder unsubstituiertes C₁-C₃₀-Alkyl, C₅₋C₃₀-substituiertes oder unsubstituiertes Cycloalkylen, substituiertes oder unsubstituiertes C₅-C₃₀-Arylen oder -Heteroarylen bedeutet,
n, m und o ganze Zahlen sind.

3. Verwendung nach Anspruch 1 und 2, worin die Piperidiniumnitroxylradikal-Einheiten enthaltenden Moleküle erhältlich sind durch Oxidation der folgenden Verbindungen 11 bis 15 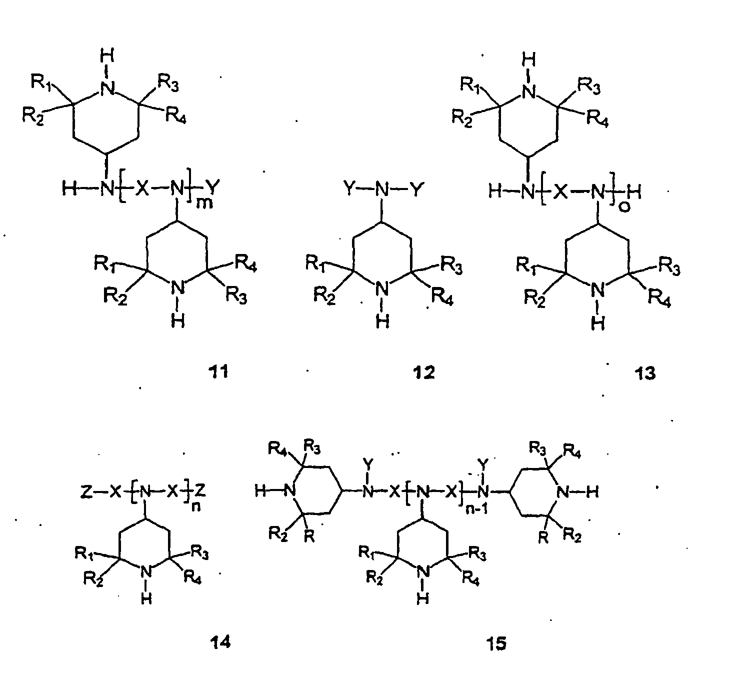 worin
R₁, R₂, R₃ und R₄ ein substituiertes oder unsubstituiertes C₁-C₁₈-Alkyl, vorzugsweise eine Methylgruppe, bedeuten,
X ein difunktionelles substituiertes oder unsubstituiertes C₂-C₃₀-Alkylen, C₅-C₃₀₋substituiertes oder unsubstituiertes Cycloalkylen, substituiertes oder unsubstituiertes C₅₋C₃₀-Arylen oder -Heteroarylen bedeutet, vorzugsweise die folgenden Strukturen 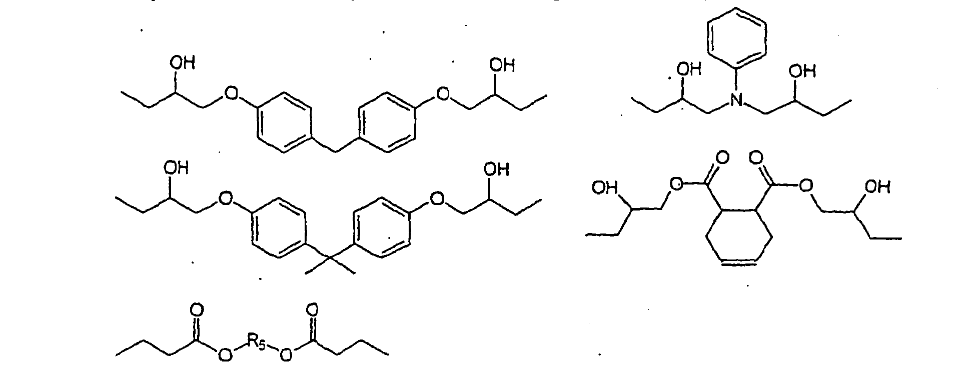
worin
R₅ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder -Heteroarylen bedeutet;
Y H oder ein monofunktionelles substituiertes oder unsubstituiertes C₁-C₁₈-Alkyl, C₅₋C₁₈-substituiertes oder unsubstituiertes Cycloalkyl, substituiertes oder unsubstituiertes C₅₋C₁₈-Aryl oder -Heteroaryl bedeutet, vorzugsweise ausgewählt aus der Gruppe 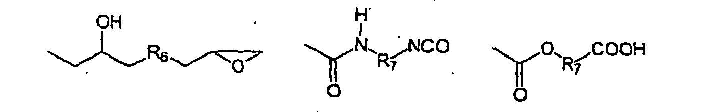 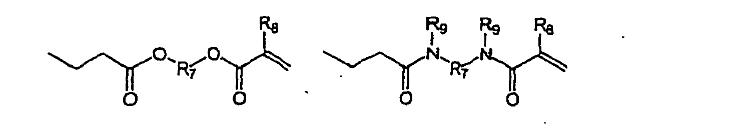
worin
R₆ ein difunktionelles substituiertes oder unsubstituiertes C₁-C₁₈-Alkylen, C₅-C₁₈₋substituiertes oder unsubstituiertes Cycloalkylen, substituiertes oder unsubstituiertes C₅₋C₁₈-Arylen oder -Heteroarylen bedeutet, und vorzugsweise 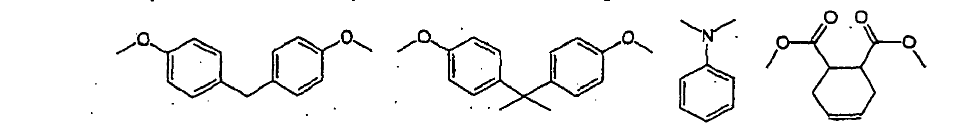
R₇ ein difunktionelles substituiertes oder unsubstituiertes C₁C₁₈-Alkylen, C₅-C₁₈₋substituiertes oder unsubstituiertes Cycloalkylen, substituiertes oder unsubstituiertes C₅₋C₁₈-Arylen oder -Heteroarylen bedeutet,
R₈ H oder ein monofunktionelles substituiertes oder unsubstituiertes C₁-C₃₀-Alkyl, C₅₋C₃₀-substituiertes oder unsubstituiertes Cycloalkylen, substituiertes oder unsubstituiertes C₅-C₃₀-Arylen oder -Heteroarylen bedeutet,
R₉ ein monofunktionelles substituiertes oder unsubstituiertes C₁-C₃₀-Alkyl, C₅-C₃₀₋substituiertes oder unsubstituiertes Cycloalkylen, substituiertes oder unsubstituiertes C₅₋C₃₀-Arylen oder -Heteroarylen bedeutet,
Z Wasserstoff bedeutet oder eine polymerisierbare Einheit, vorzugsweise ausgewählt aus der Gruppe 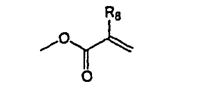
worin
R₈ H oder ein monofunktionelles substituiertes oder unsubstituiertes C₁-C₃₀-Alkyl, C₅₋C₃₀-substituiertes oder unsubstituiertes Cycloalkylen, substituiertes oder unsubstituiertes C₅-C₃₀-Arylen oder -Heteroarylen bedeutet, und n, m und o ganze Zahlen sind.

4. Verwendung nach Anspruch 1, worin die Dentalmasse Polymere, Prepolymere oder Makromonomere umfasst, die Nitroxylradikal-Einheiten enthalten, die erhältlich sind durch Umsetzen der Verbindung 16 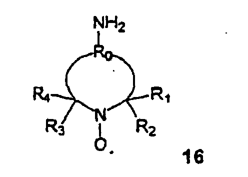 worin bedeuten
R₀ ein substituiertes oder unsubstituiertes C₁-C₁₈-Alkylen, R₁, R₂, R₃ und R₄ ein substituiertes oder unsubstituiertes C₁-C₁₈-Alkyl, vorzugsweise eine Methylgruppe,
- mit einem Molekül der Gruppe A, ausgewählt aus der Gruppe eines Diepoxids, eines Diisocyanats, einer Dicarbonsäure oder eines Derivats davon, eines Bisacrylamids oder eines Bisacrylats oder
- mit einem Molekül der Gruppe B, ausgewählt aus der Gruppe von Molekülen, die umfassen mindestens eine Epoxid- und eine Methacrylatgruppe, eine Epoxid- und eine Isocyanatgruppe, eine Methacrylat- und eine Isocyanatgruppe, eine Acrylat- und eine Methacrylatgruppe, oder
- mit einer Mischung von Molekülen A und B.

5. Verwendung nach Anspruch 4, worin Amine, die Nitroxylradikal-Einheiten enthalten, als Comonomere in Polyamiden, Polyamidoaminen, Polyesteraminen, Polyharnstoffen, Epoxid-Amin-Additionspolymeren enthalten sind.

6. Verwendung nach Anspruch 4, worin Amine, die Nitroxylradikal-Einheiten enthalten, als Comonomere in Makromonomeren oder Prepolymeren enthalten sind, die Polyamid, Polyamidoamin, Polyesteramin, Polyharnstoff oder Epoxid-Amin-Additionspolymer-Struktureinheiten aufweisen.

7. Verwendung nach Anspruch 1, worin Moleküle, die Piperidiniumnitroxylradikal enthalten, Verbindungen 17 und 18 sind

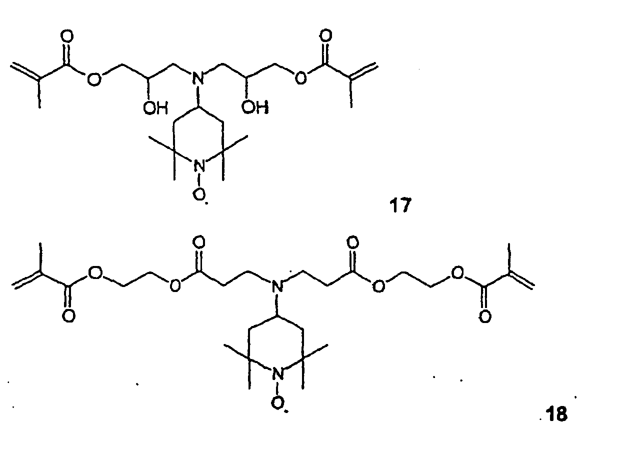

8. Verwendung nach Anspruch 1, worin die Dentalmasse stabile Radikale der Formel 1 bis 5 in einer Menge von 0,001 bis 3,0 Gew.-% umfassen.

9. Verwendung nach Anspruch 1, worin die Dentalmasse stabile Radikale der Formel 1 bis 5 in einer Menge von 0,01 bis 1,0 Gew.-% umfassen.

10. Verwendung nach Anspruch 1, worin die Dentalmasse stabile Radikale der Formel 1 bis 5 in einer Menge von 0,01 bis 0,2 Gew.-% umfassen.

11. Verfahren zur Bereitstellung einer Dentalmasse mit Licht- und thermischer Stabilität, das umfasst das Einarbeiten eines Prepolymers, Makromonomers oder Polymers, das eine 4-Amino-2,2,6,6-tetramethylpiperidin-1-oxyl-Einheit aufweist, in die Dentalmasse.

## Revendications

1. Utilisation d'au moins l'un des radicaux stables de formules 1 à 5 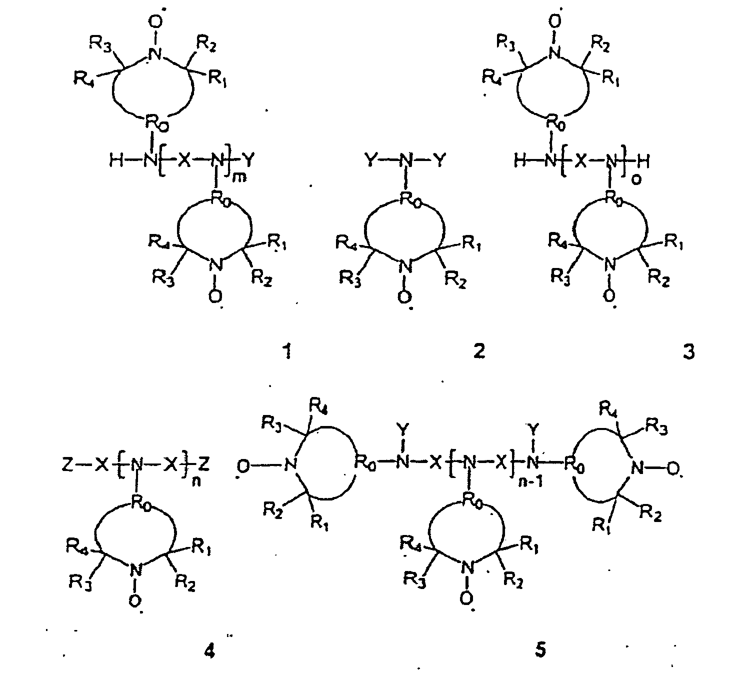 où
R₀ désigne un groupe alkylène en C₁ à C₁₈ substitué ou non substitué,
R₁, R₂, R₃ et R₄ désignent un groupe alkyle en C₁ à C₁₈ substitué ou non substitué ;
X désigne un groupe difonctionnel alkylène en C₂ à C₃₀ substitué ou non substitué, cycloalkylène en C₅ à C₃₀ substitué ou non substitué, arylène ou hétéroarylène en C₅ à C₃₀ substitué ou non substitué, de préférence les structures suivantes 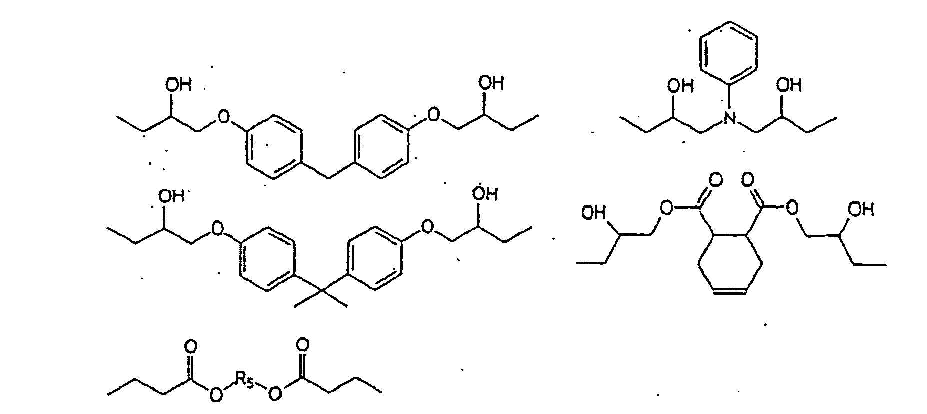
où
R₅ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué,
Y désigne H ou un groupe monofonctionnel alkyle en C₁ à C₁₈ substitué ou non substitué, cycloalkyle en C₅ à C₁₈ substitué ou non substitué, aryle ou hétéroaryle en C₅ à C₁₈ substitué ou non substitué, de préférence choisi dans le groupe 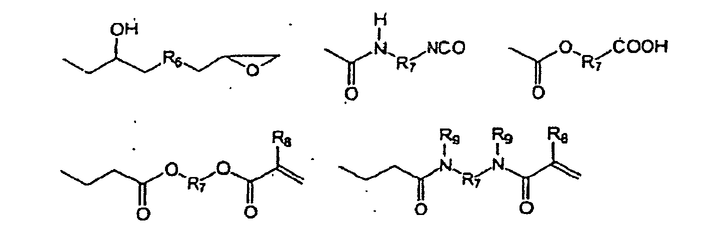
où
R₆ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué, de préférence 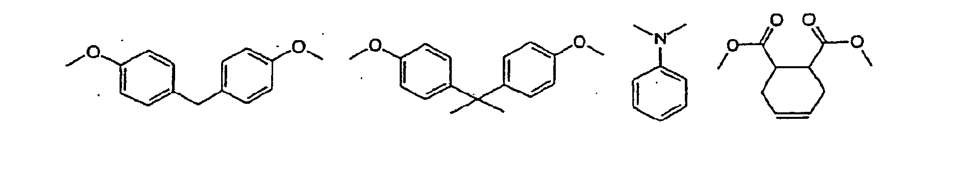
R₇ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué,
R₈ désigne H ou un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué,
R₉ désigne un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué,
Z désigne l'hydrogène, ou un fragment polymérisable, de préférence choisi dans le groupe de 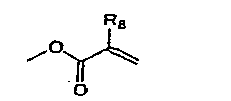
où
R₈ désigne H ou un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué,
n, m et o sont des nombres entiers,
dans la fabrication d'une composition dentaire douée de stabilité à la lumière et à la chaleur.

2. Utilisation selon la revendication 1, dans laquelle la composition dentaire comprend au moins l'un des composés 6 à 10 qui a au moins un fragment à radical nitroxyle de pipéridinium 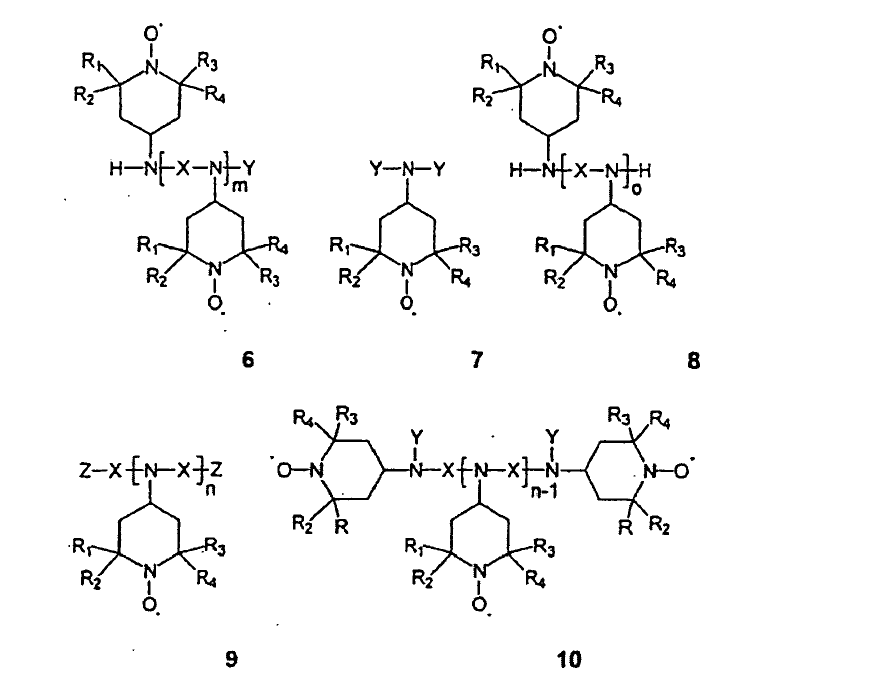 où
R₁, R₂, R₃ et R₄ désignent un groupe alkylène en C₁ à C₁₈ substitué ou non substitué, de préférence un groupe méthyle ;
X désigne un groupe difonctionnel alkylène en C₂ à C₃₀ substitué ou non substitué, cycloalkylène en C₅ à C₃₀ substitué ou non substitué, arylène ou hétéroarylène en C₅ à C₃₀ substitué ou non substitué, de préférence les structures suivantes 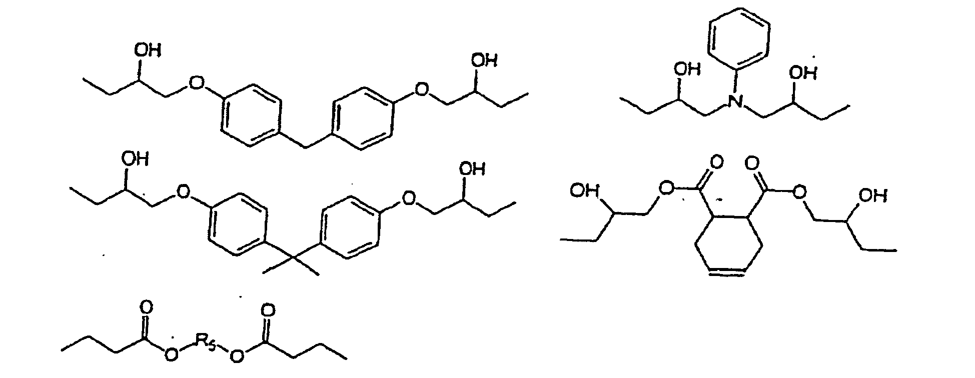
où
R₅ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué,
Y désigne H ou un groupe monofonctionnel alkyle en C₁ à C₁₈ substitué ou non substitué, cycloalkyle en C₅ à C₁₈ substitué ou non substitué, aryle ou hétéroaryle en C₅ à C₁₈ substitué ou non substitué, de préférence choisi dans le groupe 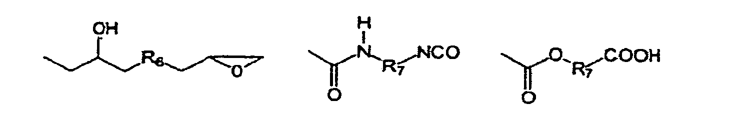 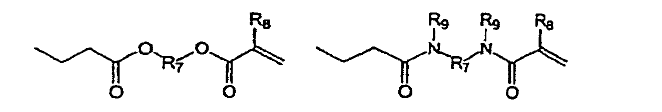
où
R₆ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué, de préférence 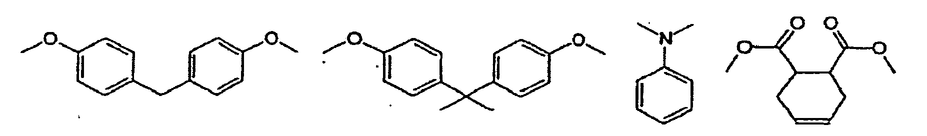
R₇ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué,
R₈ désigne H ou un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué
R₉ désigne un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué
Z désigne l'hydrogène ou un fragment polymérisable, de préférence choisi dans le groupe de 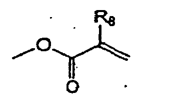
où
R₈ désigne H ou un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué, n, m et o sont des nombres entiers.

3. Utilisation selon les revendications 1 et 2, dans laquelle les molécules contenant des fragments à radical nitroxyle de pipéridinium peuvent être obtenues par oxydation des composés 11 à 15 suivants 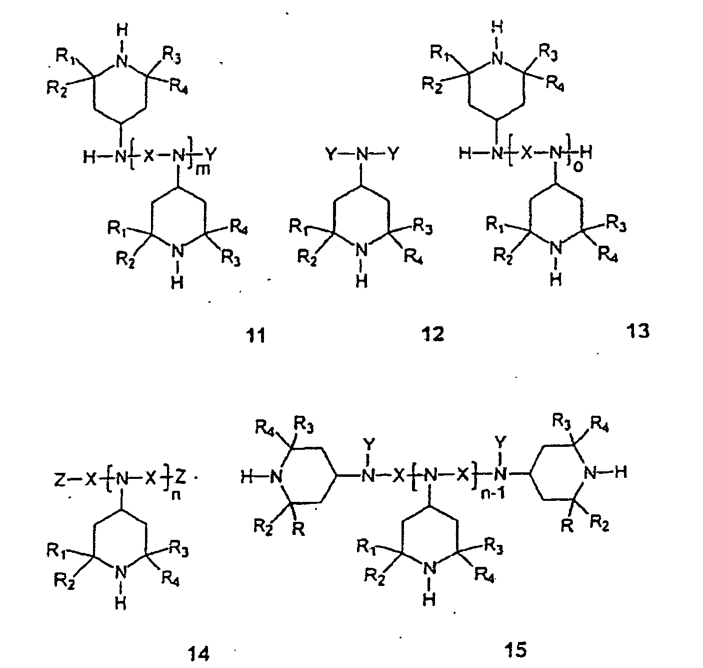 ou
R₁, R₂, R₃ et R₄ désignent un groupe alkylène en C₁ à C₁₈ substitué ou non substitué, de préférence un groupe méthyle
X désigne un groupe difonctionnel alkylène en C₂ à C₃₀ substitué ou non substitué, cycloalkylène en C₅ à C₃₀ substitué ou non substitué, arylène ou hétéroarylène en C₅ à C₃₀ substitué ou non substitué, de préférence les structures suivantes 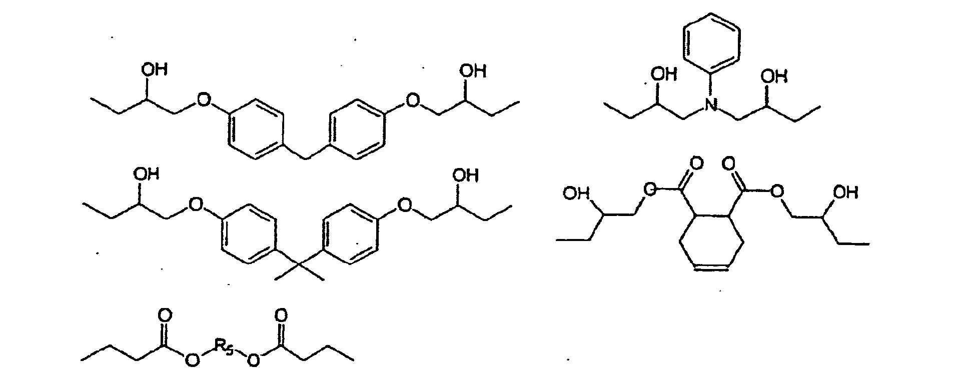
où
R₅ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué,
Y désigne H ou un groupe monofonctionnel alkyle en C₁ à C₁₈ substitué ou non substitué, cycloalkyle en C₅ à C₁₈ substitué ou non substitué, aryle ou hétéroaryle en C₅ à C₁₈ substitué ou non substitué, de préférence choisi dans le groupe 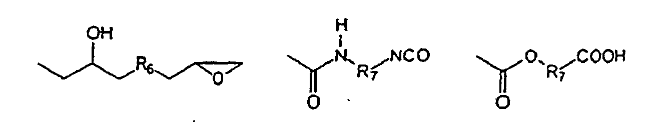 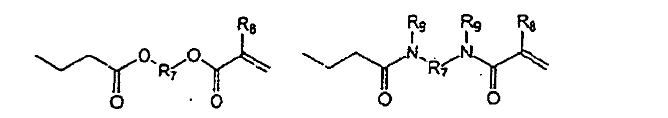
où
R₆ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué, de préférence 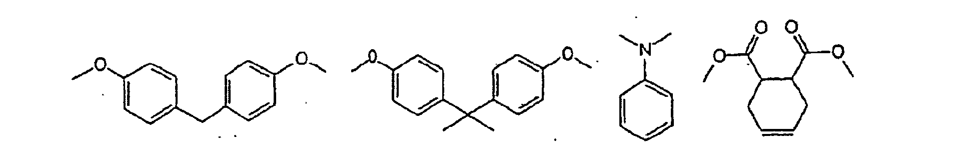
R₇ désigne un groupe difonctionnel alkylène en C₁ à C₁₈ substitué ou non substitué, cycloalkylène en C₅ à C₁₈ substitué ou non substitué, arylène ou hétéroarylène en C₅ à C₁₈ substitué ou non substitué,
R₈ désigne H ou un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué
R₉ désigne un groupe monofonctionnel alkylène en C₁ à C₃₀ substitué ou non substitué, cycloalkylène en C₅ à C₃₀ substitué ou non substitué, arylène ou hétéroarylèneen C₅ à C₃₀ substitué ou non substitué
Z désigne l'hydrogène, ou un fragment polymérisable, de préférence choisi dans le groupe de 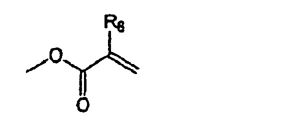
où
R₈ désigne H ou un groupe monofonctionnel alkylène en C₁ à C₃₀ substitué ou non substitué, cycloalkylène en C₅ à C₃₀ substitué ou non substitué, arylène ou hétéroarylène en C₅ à C₃₀ substitué ou non substitué,
n, m et o sont des nombres entiers.

4. Utilisation selon la revendication 1, dans laquelle la composition dentaire comprend des polymères, prépolymères ou macromonomères contenant des fragments à radical nitroxyle, qui peuvent être obtenus par réaction du composé 16 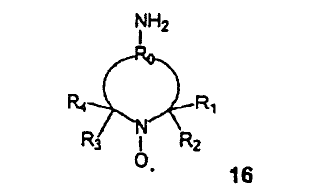 où
R₀ désigne un groupe alkylène en C₁ à C₁₈ substitué ou non substitué, R₁, R₂, R₃ et R₄ désignent un groupe alkylène en C₁ à C₁₈ substitué ou non substitué, de préférence un groupe méthyle
- avec une molécule du groupe A, choisie dans le groupe formé par un diépoxyde, un diisocyanate, un acide dicarboxylique ou un dérivé de celui-ci, un bisacrylamide ou un bisacrylate ou
- avec une molécule du groupe B, choisie dans le groupe formé par les molécules qui comprennent au moins un époxyde et un groupe méthacrylate, un époxyde et un isocyanate, un méthacrylate et un groupe isocyanate, un acrylate et un groupe méthacrylate, ou
- avec un mélange de molécules A et B.

5. Utilisation selon la revendication 4, dans laquelle des amines contenant des fragments à radical nitroxyle sont contenues en tant que comonomères dans des polyamides, polyamidoamines, polyesteramines, polyurées, polymères d'addition époxyde-amine.

6. Utilisation selon la revendication 4, dans laquelle des amines contenant des fragments à radical nitroxyle sont contenues en tant que comonomères dans des macromonomères ou prépolymères ayant des motifs structuraux de polyamide, polyamidoamine, polyesteramine, polyurée ou polymère d'addition époxyde-amine.

7. Utilisation selon la revendication 1, dans laquelle les molécules contenant un radical nitroxyle de pipéridinium sont les composés 17 et 18.

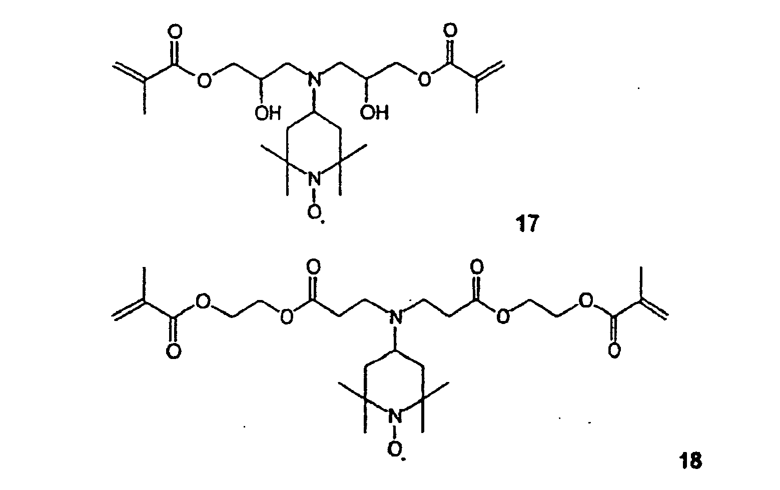

8. Utilisation selon la revendication 1, dans laquelle la composition dentaire comprend des radicaux stables de formules 1 à 5 en une proportion de 0,001 à 3,0 % en poids.

9. Utilisation selon la revendication 1, dans laquelle la composition dentaire comprend des radicaux stables de formules 1 à 5 en une proportion de 0,01 à 1,0 % en poids.

10. Utilisation selon la revendication 1, dans laquelle la composition dentaire comprend des radicaux stables de formules 1 à 5 en une proportion de 0,01 à 0,2 % en poids.

11. Procédé pour conférer de la stabilité à la lumière et à la chaleur à une composition dentaire, qui comprend l'incorporation dans la composition dentaire d'un prépolymère, macromonomère ou polymère ayant un fragment 4-amino-2,2,6,6-tétraméthylpipéridine-1-oxyle.
